# EUROPEAN PATENT SPECIFICATION

(11) **EP 1 968 679 B1**
(45) Date of publication and mention of the grant of the patent: **21.09.2016**
(21) Application number: 06840297.3
(22) Date of filing: 18.12.2006
(51) Int. Cl.: A61B 18/14, A61M 25/01

(54) **DEFLECTABLE CATHETER WITH A FLEXIBLY ATTACHED TIP SECTION**
ABLENKBARER KATHETER MIT FLEXIBEL BEFESTIGTEM SPITZENABSCHNITT
CATHETER A DEFLEXION AVEC UNE PARTIE D'EXTREMITE FIXEE DE MANIERE FLEXIBLE

(30) Priority: 29.12.2005 US 323908
(43) Date of publication of application: 17.09.2008
(73) Proprietor: Biosense Webster, Inc., Diamond Bar, CA 91765 (US); Worley, Seth J., Lancaster, PA 17603 (US)
(72) Inventor: WORLEY, Seth, J., Lancaster, PA 17603 (US); SHARAREH, Shiva, Laguna Niguel, CA 92677 (US)
(74) Representative: Small, Gary James
(86) International application number: PCT/US2006/062215
(87) International publication number: WO 2007/076312

(56) References cited:
- WO-A1-2007/035554
- US-A- 5 617 854
- US-A- 5 782 239
- US-A- 5 931 811
- US-A- 6 002 955
- US-A1- 2003 105 453
- US-A1- 2003 125 720
- US-A1- 2005 015 082
- US-A1- 2005 267 459
- US-B2- 6 922 579

## Description

### FIELD OF THE INVENTION

The invention is directed to a catheter having a tip assembly for mapping and/or ablating regions of or near a heart.

### BACKGROUND OF THE INVENTION

For successfully mapping and/or ablating of regions of or near a heart, the tip assembly should ideally make contact with the surface of the heart without undue pressure. Excess pressure can result in mechanical trauma and damage to the heart and/or result in inadequate cooling of the tip of the catheter via the blood stream resulting in steam pops, char, coagulation, embolization and inadequate delivery of current for successfully ablation of the tissue. Catheter-based ablation is usually conducted within the heart. The inside of the heart is a complex three-dimensional structure with both concave, convex and tubular structures as well as multiple irregularities within the convex or on the concave structure. Further, the transition from concave to convex or into a tubular structure also results in changes in the surface contour of the inside of the heart. Depending on the mechanism of the cardiac arrhythmia, ablation may be required within a concave structure with both smooth and irregular surface contours, on a convex structure with both smooth and irregular surface contours or at the intersection of two or more complex structures. Ablation may also be required within, around and on complex three-dimensional contours created by the confluence of a concave, convex and a tubular structure which themselves may have smooth or irregular contours. Currently, available catheter technology attempts to address ablation of these various areas of the heart with an ablation tip assembly, the shape and direction of which is determined by puller wires or preset shapes where the bending modulus between the mapping or ablation section and the intermediate section is constant. The ability of the ablation section to accommodate the irregular contours within the heart is limited. Attempts to approximate and contact these complex surface contours with the ablation section may result in either no contact or excess surface pressure at the tip allow the ablation section to achieve the off axis angle from the intermediate section required to make surface contact.

A catheter design with an ablation or mapping tip assembly attached to the intermediate section by a flexible section with a modulus of elasticity that allows the tip assembly to be deflected without displacing the intermediate section of the catheter is important to successful and safe ablation. Further, it is recognized by one of ordinary skill in the art that specific arrhythmias associated with defined surface contours may be optimally addressed with a catheter where the flexible section connecting the intermediate section and the mapping and ablation assembly is off set from the intermediate section at a predefined angle either in the plane or out of the plane of the intermediate section.

Specific examples are provided below:
Atrial flutter and atrial fibrillation are common sustained cardiac arrhythmias. Atrial flutter occurs when the atria are stimulated to depolarize at 200-350 beats per minute and is maintained by macroreentrant circuits generated by electrical impulses traveling in a circular fashion around and in the atria. Atrial flutter results in poor atrial pumping since some parts of the atria are releasing while other parts are contracting. Fortunately, atrial flutter in the right atrium can be effectively treated by ablation of the inferior vena cava - tricuspid annulus isthmus to create a line of conduction block to interrupt the macroreentrant circuit. The region at or near the inferior vena cava - tricuspid annulus isthmus (hereinafter referred to as "the cava-tricuspid region") can be difficult to map or ablate. Not only does the tissue in that region have a convex curvature contrary to the generally cavernous shape of the right atrium, the tissue surface is uneven. Therefore, it is desirable for a catheter entering the right atrium from the inferior vena cava (an entry that is below or inferior of the cava-tricuspid region) to have a catheter body that can be deflected to approximate the convex curvature of the cava-tricuspid region and a preshaped flexible off-axis catheter tip in the direction of deflection that can maintain contact with the uneven tissue as the tip is dragged along for mapping or ablation procedures.

To successfully ablate other ventricular and atrial arrhythmias, a focal lesion or a line of conduction block should be created in the generally concave cavity of the right atrium/left atrium/right ventricle/left ventricle (RA/LA/RV/LV). The tissue surface of these structures is generally uneven. Therefore, it is generally desirable to have a catheter body that can be deflected to approximate the concave curvature of the region and a pre-shaped flexible off-axis in-plane catheter tip that is opposite to the direction of deflection that can maintain contact with the uneven tissue as the tip is dragged along for mapping or ablation procedures.

In patients with refractory atrial fibrillation, the atria are stimulated to depolarize irregularly at 250-400 cycles per minute. Not every atrial activation results in a QRS complex (ventricular depolarization) because the AV Node acts as a filter. However, there are instances where it is desirable to create conduction block at or near the AV Bundle. This region of the right atrium, the atrioventricular Bundle (of His) near the Atrioventricular (AV) Node, poses similar challenges for mapping and ablation as the cava-tricuspid region. The region is also convex unlike the generally cavernous contour of the right atrium. Moreover, the atrium wall in this region is canted slightly to the anterior. Therefore, it is desirable for a catheter entering from the inferior vena cava (an entry that is also below or inferior of AV Bundle) to have a catheter body that can be deflected to approximate the convex curvature of the region and a preshaped flexible catheter tip that extends off-plane from the catheter body to circumvent the canted angle of tissue surface.

As with most catheter-based mapping and/or ablation procedures, the catheter section immediately proximal the tip may not be in contact with or supported/stabilized by any structure in the heart. Without supportive contact between this proximal catheter section and the tissue, motion of the heart during systole, diastole and respiration is not transmitted to this catheter section except by contact between tissue and the catheter tip. As the heart moves during systole, diastole and respiration, the contact pressure at the tip of the catheter may vary from excessive to nonexistent. In a catheter that approaches the atrium in a "forward" direction, the disparity between the generally motionless (or out of synch) catheter and the heart can make it difficult to maintain stable contact between the catheter tip and the atrium wall in a beating moving heart. An unsupported and thus unsynchronized catheter used in the atrium may be inadvertently advanced into the tricuspid valve. Also, nonuniform contours in the atrium can make it difficult to contact recessed areas without excess pressure on the protruding areas increasing the risk of perforation. In addition, the catheter position is maintained only by contact between the tip and the nonuniform contours causing the catheter tip to frequently lose contact with the tissue during ablation or mapping as the heart moves independently during systole, diastole and with respiration.

Accordingly, a desire exists for a catheter capable of effectively mapping and ablating complex regions such as those with a convex contour, such as the cava-tricuspid region and regions at or near the AV Bundle (of His). It is desirable that the catheter body is adapted to approximate the convex contour for improved access to the tissue of interest from the inferior vena cava, and that the catheter tip be able to maintain contact with the tissue surface without undue force and maintain stability during ablation and mapping despite the motion of beating heart in a breathing patient. A catheter of such design improves precision of mapping and/or ablation and minimises risks of damage to the tissue, including tissue perforation and inadvertent entry into the tricuspid valve.

WO2007/035554 discloses a catheter for mapping and/or ablating continuous linear or circumferential lesions at the intersection of a generally flat structure, such as the left atrium, and the ostium of generally cavernous regions of the heart, including pulmonary vein and the pulmonary venous antrum, and comprises a catheter body with an intermediate section that is connected to a tip assembly by a highly flexible section. US5931811 discloses a steerable catheter including a complexly curved proximal section shaped to seat the catheter relative to an anatomical feature within a patient. The catheter also includes a flexible intermediate section which has comparatively greater flexibility than other section of the catheter. US2003/0125720 discloses an ablation instrument having a flexible portion at or near the distal portion of the instrument. The instrument includes an elongated tubular member having a steerable distal end configured to deflect or otherwise direct and properly position at least a portion of the distal portion, comprising an ablation device, during an ablation procedure. US2005/0015082 discloses a catheter for ablating tissue, comprising an elongated generally-tubular body having distal and proximal ends. An electrode assembly is provided at the distal end of the catheter body. US5617854 discloses a pre-shaped cardiac catheter for mapping and selective ablation of a portion of cardiac circuitry, including a pre-shaped first curved portion for positioning around the ostium of coronary sinus and a second curved portion for maintaining the first curved portion in its desired position. US6002955 discloses a stabilised electrophysiology catheter including a main body portion and a flexible tip portion. A plurality of electrodes are positioned along the tip portion.

### SUMMARY OF THE INVENTION

The present invention is directed to a catheter as defined in claim 1, and comprises a catheter body with an intermediate section that is connected to a tip assembly by a highly flexible pre-shaped section. The entire intermediate section may be deflected, or the intermediate section may comprise a deflectable proximal portion and a distal portion that is straight or curved. The highly flexible section presets the tip assembly at an off-plane angle from the intermediate section.

The intermediate section may have a distal portion with shape memory to maintain a straight configuration or a curved configuration to improve approximation to the generally convex regions of the cavo-tricuspid isthmus and His regions or the generally concave regions of the RA/RA/LA/LV.

A high bending modulus of the flexible section that connects the tip section for mapping and ablation to the intermediate section enables the flexible section to absorb displacement force applied to the tip assembly, without displacing the intermediate section improving tissue contact when the tip assembly encounters uneven tissue surface. The high bending modulus of the flexible section allows the tip section to be displaced while limiting the force that the tip assembly can apply to the tissue reducing the risk of any of the following: direct mechanical perforation, steam pop perforation, and burying of the tip assembly in the myocardium resulting in high temperatures, low energy delivery, thrombus and char formation.

The specific application of the catheter of the present invention determines how the flexible section connects the tip assembly to the intermediate section. Parameters of the flexible section that determine the relationship between the tip assembly and the intermediate section includes the following:
a) the off plane and/or off axis angle of the flexible section to the intermediate section, b) the flexibility of the flexible section c) the lateral stability of the flexible section, and d) the length of the flexible section.

In addition, the configuration of the intermediate section to which the tip assembly is flexibly attached also impacts on the function of the tip assembly. As mentioned, the entire intermediate section may be deflectable, or only its proximal section from which a straight or curved distal section extends. In addition, how the tip assembly flexibly extends in relation to the straight or curved distal section of the intermediate section also determines the specific application. The tip assembly can be flexibly attached out of plane with any curved distal section,, the length of the tip assembly beyond the flexible section, and the construction of the tip assembly beyond the flexible section (e.g., irrigated or irrigated with or without temperature sensors or electromagnetic sensors) can be varied. In one embodiment, the present invention is directed to a catheter configured for mapping and ablating a generally convex region of the heart, such as the complex intersection of the inferior vena cava, RA, and RV at the cavo-tricuspid isthmus. In a detailed embodiment, the catheter has an intermediate section and a tip assembly adapted for mapping and/or ablation that is attached to the intermediate section by a pre-shaped flexible section that allows the tip assembly to be moved generally independently of the intermediate section. The catheter comprises an elongated flexible tubular body having proximal and distal ends. The intermediate section is mounted on the distal end of the tubular body and deflected with a curvature that approximates t he generally convex contour of the cava-tricuspid isthmus or Bundle of His region of the right atrium. The tip assembly is attached to the end of the intermediate section by the flexible section which is configured with preset angles to extend the tip assembly off-plane from the intermediate section so that the tip assembly can make suitable contact with the tissue surface of the isthmus and His region.

When deflected, the intermediate section of the catheter is configured to conform to the generally convex region so that motion of the heart is transferred to the catheter thereby providing stability to the tip assembly. The preshaped flexible section improves the ability of the tip assembly to access, contact and remain in contact with surrounding tissues of variable contour without undue pressure. Moreover, the preshaped flexible section may be reinforced to provide the tip assembly with stability in a selected angle. Accordingly, the catheter of the present invention has improved safety features and improved ablation and mapping capabilities.

In another embodiment the tip assembly is configured as an ablation assembly that may be irrigated, comprising a plurality of irrigation ports in between which an ablation coil electrode is wound. A porous covering, preferably made of expanded polytetrafluoroethylene, covers the coil electrode and irrigation ports. Fluid passes through the irrigation ports to the porous covering, which then disperses the fluid around the ablation assembly. This irrigation generally enables the creation of deeper lesions.

In use, the distal end of the catheter is inserted into a patient's body and advanced atraumatically into the right atrium of a patient's heart by entry from the inferior vena cava. The intermediate section is deflected onto or near a generally convex such as the cava-tricuspid isthmus or the His region. The off-plane angle of the tip assembly readily allows the tip assembly to contact the His region notwithstanding the awkward angle imposed on the catheter by the relative superior and/or anterior locations of these regions of interest relative to the inferior vena cava.

As the user operates the catheter and maneuvers the tip assembly, the deflected intermediate section advantageously synchronizes the catheter and the tip assembly with the motion of the heart while the pre-shaped flexible section advantageously allows the tip assembly to flex from the preset angle(s) as needed in order to remain in contact with the tissue. In one embodiment, as the tip assembly encounters protrusions and recesses while being dragged along the tissue surface, the tip assembly is jarred from its preset angle but the flexible section allows the tip assembly to conform and ride along on the uneven surface without displacing the intermediate section.

By adjusting the preset angles of the flexible section, the off-plane angle of the tip assembly the catheter can be adapted to ablate and/or map most if not all convex regions in the right atrium. Accordingly, improved focal and linear ablation and mapping can be accomplished with the catheter of the present invention despite convex contour or uneven tissue surface.

In another embodiment, the present invention is directed to a catheter configured for mapping and ablation a generally concave or tubular region of the heart, such as the cavity of the RA, RV, LA, LV, rVC or SVC or other tubular structures. In a detailed embodiment, the catheter has an intermediate section and a tip assembly adapted for mapping and/or ablation that is attached to the intermediate section by a pre-shaped flexible section that allows the tip assembly to be moved generally independently of the intermediate section. The catheter comprises an elongated flexible tubular catheter body having proximal and distal ends. The intermediate section is mounted on the distal end of the tubular body and deflected with a curvature that approximates the generally concave contour of the cavitary or tubular structure. The tip assembly is attached to the end of the intermediate section by the flexible section which is configured with preset angles to extend the tip assembly off-plane from the intermediate section so that the tip assembly can make suitable contact with the tissue surface of the cavitary or tubular structure When deflected, the intermediate section of the catheter is configured to conform to the generally concave or tubular region so that motion of the heart is transferred to the catheter thereby providing stability to the tip assembly. The preshaped flexible section improves the ability of the tip assembly to access, contact and remain in contact with surrounding tissues of variable contour without undue pressure. Moreover, the preshaped flexible section may be reinforced to provide the tip assembly with stability in a selected angle. Accordingly, the catheter of the present invention has improved safety features and improved ablation and mapping capabilities. In another embodiment, of the concave design the tip assembly is configured as an ablation assembly that may be irrigated, comprising a plurality of irrigation ports in between which an ablation coil electrode is wound. A porous covering, preferably made of expanded polytetrafluoroethylene, covers the coil electrode and irrigation ports. Fluid passes through the irrigation ports to the porous covering, which then disperses the fluid around the ablation assembly. This irrigation generally enables the creation of deeper lesions. In use, the distal end of the catheter is inserted into a patient's body and advanced atraumatically into cavity or tubular structure. The intermediate section is deflected onto or near a generally concave structure such as the RA, RV, LA, LV, SVC or IVC or other cavitary or tubular structures. The angle of the tip assembly readily allows the tip assembly to contact the surface notwithstanding the awkward angle imposed on the catheter by surface irregularities.

As the user operates the catheter and maneuvers the tip assembly, the deflected intermediate section advantageously synchronizes the catheter and the tip assembly with the motion of the heart while the pre-shaped flexible section advantageously allows the tip assembly to flex from the preset angle(s) as needed in order to remain in contact with the tissue. In one embodiment, as the tip assembly encounters protrusions and recesses while being dragged along the tissue surface, the tip assembly is jarred from its preset off axis angle but the flexible section allows the tip assembly to conform and ride along on the uneven surface without displacing the intermediate section.

By adjusting the preset angles of the flexible section, the off-plane angles of the tip assembly the catheter can be adapted to ablate and/or map most if not all concave regions. Accordingly, improved focal and linear ablation and mapping can be accomplished with the catheter of the present invention despite concave contour or uneven tissue surface.

### BRIEF DESCRIPTION OF THE DRAWINGS

These and other features and advantages of the present invention will be better understood by reference to the following detailed description when considered in conjunction with the accompanying drawings, wherein:
**FIG. 1** is an elevated side view of an exemplary catheter where the flexible section is pre-shaped at an angle in the same direction as the deflection of the intermediate section;
**FIG. 1A** is a schematic perspective view of the distal end of the intermediate section, the flexible section and the tip assembly of the catheter of **FIG. 1** in use at or near a generally convex region of the right atrium, such as a cava-tricuspid isthmus;
**FIG. 2** is an elevated side view of another exemplary catheter where the flexible section is preshaped at an angle opposite to the direction of deflection of the intermediate section;
**FIG. 2a** is a side cross-sectional view of a catheter body according to the catheter of **FIG.1****,** including the junction between the catheter body and the intermediate section;
**FIG. 2b** is a side cross sectional view taken of the side opposite that of **FIG. 2a** of the catheter body of **FIG. 2a****,** including the junction between the catheter body and the intermediate section;
**FIG. 3** is a side cross sectional view of the intermediate section of the catheter of **FIG. 1****,** including the junction between the intermediate section and the flexible section;
**FIG. 3a** is a longitudinal cross sectional view of the intermediate section of **FIG. 3** taken along line 3a-3a;
**FIG. 3b** is a side cross sectional view of the flexible section of the catheter of **FIG. 1****,** including the junction between the flexible section and the tip assembly;
**FIG. 3c** is a longitudinal crosssection view of the flexible section of **FIG. 3** taken along line 3d-3d;
**FIG. 4** is an enlarged side view of the distal end of the intermediate section, the flexible section and the tip assembly of **FIG. 1****;**
**FIG. 5** is a top view of the intermediate section, the flexible section and the tip assembly of an embodiment of the catheter of the present invention, with the flexible section preset to support the tip assembly off plane with the intermediate section;
**FIG. 5A** is a schematic perspective view of the intermediate section, the flexible section and the tip assembly of the catheter of **FIG. 5** in use at or near a generally convex region of the right atrium, such as a Bundle of His;
**FIG. 5b** is a schematic perspective view of the distal end of the intermediate section, the flexible section and the tip assembly of the catheter of **FIG. 2** in use at or near a generally concave region such as the RA, RV, LA, LV;
**FIG. 5c** is a schematic perspective view of the distal end of the intermediate section, the flexible section and the tip assembly of the catheter of **FIG. 2** in use at or near a generally tubular region such as the SVC or IVC;
**FIG. 5d** is a schematic perspective view of the distal end of the intermediate section, the flexible section and the tip assembly of the catheter of **FIG. 1** in use at or near a generally concave region such as the RA, RV, LA LV;
**FIG. 5e** is a schematic perspective view of the distal end of the intermediate section, the flexible section and the tip assembly of the catheter **of** **FIG.1** in use at or near a generally tubular region such as the SVC or IVC;
**FIG. 6a** is a close-up side view of an embodiment of an irrigated ablation assembly; and
**FIG. 6b** is a close-up longitudinal cross-sectional view of the ablation assembly depicted in **FIG. 5A** taken along line 5b-5b;
**FIG. 7** is a side view of an embodiment of a catheter body, a deflectable intermediate section, and a tip assembly connected by a flexible section that extends the tip assembly in an off-axis direction generally opposite to the direction of deflection;
**FIG. 8** is a side view of an embodiment of a catheter body, an intermediate section with a deflectable proximal section and a generally linear distal section, and a tip assembly connected by a flexible section that extends the tip assembly in an off-axis direction generally in the same direction as the direction of deflection; and
**FIG.** 9 is a side view of an embodiment of a catheter body, an intermediate section with a deflectable proximal section and a curved distal section, and a tip assembly connected by a flexible section that extends the tip assembly in an off-axis direction generally opposite to the direction of deflection.

### DETAILED DESCRIPTION OF THE INVENTION

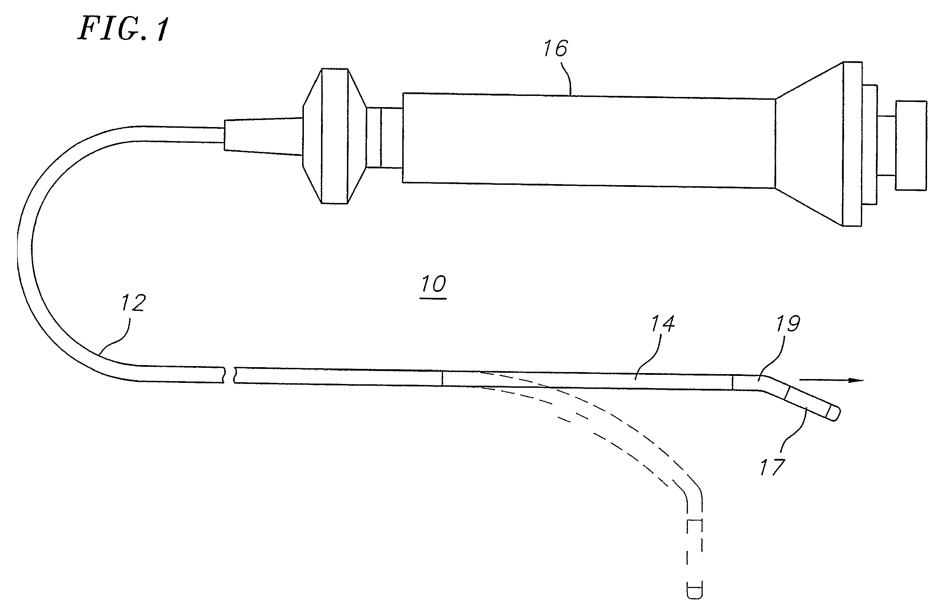

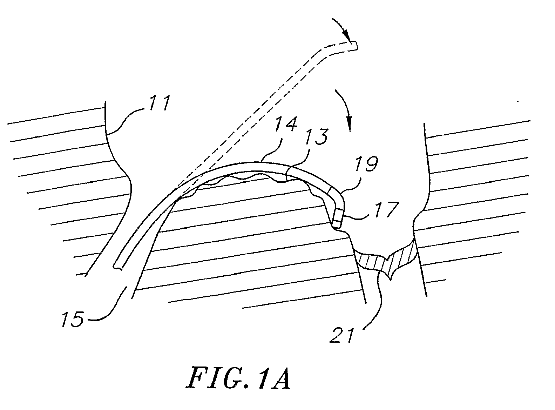

Referring to FIG. 1, the present disclosure provides a catheter **10** having a tip assembly **17** for mapping and/or ablation at its distal end. The catheter comprises an elongated catheter body **12** having proximal and distal ends, a deflectable intermediate section **14** at the distal end of the catheter body **12,** and a control handle **16** at the proximal end of the catheter body. The tip assembly **17** is connected to the deflectable intermediate section **14** by a flexible section **19** which enables the tip assembly **17** to extend from the intermediate section **14** either in plane with or at a preset off-axis angle and/or off-plane angle. In the illustrated example, the tip assembly **17** is adapted for ablation although it is understood by one of ordinary skill in the art that the tip assembly may be adapted for mapping applications, as well.

Referring to **FIG.** 1A, the catheter **10** is adapted for use in a right heart
**11** to map or ablate a region with a generally convex contour such as an inferior vena cava-tricuspid isthmus **13.** Advantageously, this region is accessible to the catheter **10** despite the catheter's entry to the atrium from an inferior vena cava **15** and the catheter's forward approach to the isthmus treatment site. In particular, the intermediate section **14** is deflected so the tip assembly can reach the isthmus despite the generally convex curvature of the isthmus. The deflection also enables the intermediate section to approximate and assume the convex curvature such that motion of the heart.is transferred to catheter to stabilize the catheter. Moreover, notwithstanding the relatively acute and awkward angle of the isthmus encountered by the catheter extending from the inferior vena cava, contact between the tip assembly 17 and tissue surface of the isthmus **13** is enabled by an in-plane canting of the tip assembly in the direction of the deflection. The highly flexible section **19** connecting the tip assembly 17 and the intermediate section **14** not only enables the in-plane extension of the tip assembly but it also allows the tip assembly to maintain contact with the tissue surface despite the uneven and nonuniform surface of the isthmus **13** that spans between a tricuspid valve **21** and the inferior vena cava IS which has recesses and protrusions that are encountered by the tip assembly **17** as it is dragged along to map and/or ablate the isthmus.

The flexible section **19** is preshaped with a configuration that attaches the tip assembly **17** of this embodiment at a predetermined off-axis angle relative to the intermediate section **14** in a direction of the deflection of the intermediate section. Moreover, the flexible section **19** has a bending modulus greater than that of the intermediate section **14** so the tip assembly **17** can flex and adjust to the contour of the isthmus tissue surface independently of the intermediate section **14.** As shown in **FIG. 1A****,** the off-axis extension of the tip assembly **17** from the intermediate section **14** enables contact between the tip assembly 17 and tissue surface of the isthmus. The ability of the tip assembly to flex and adjust permits the tip assembly **17** to contact tissue in recessed areas without exerting excess contact pressure in elevated areas reducing the risk of perforation.

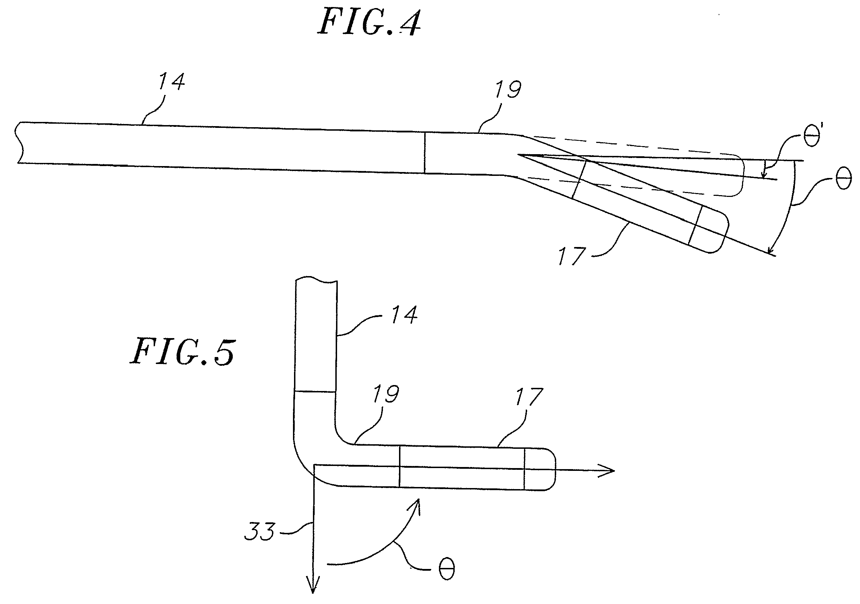

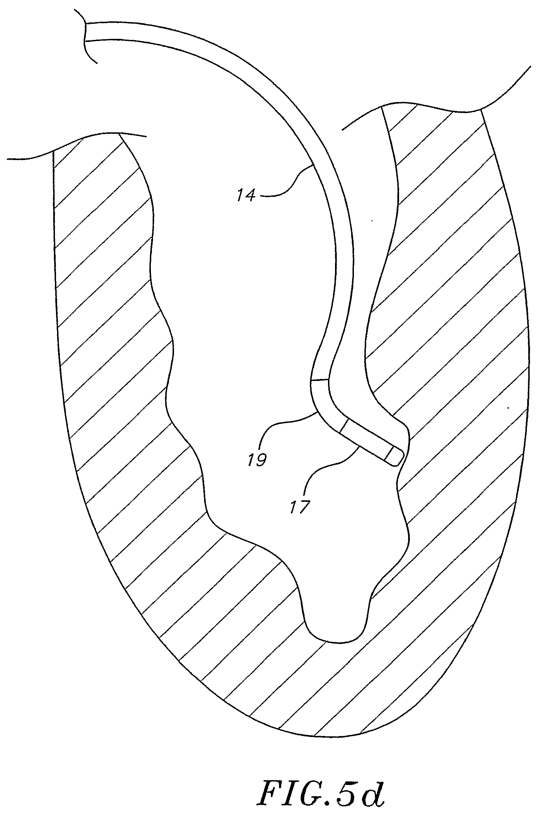

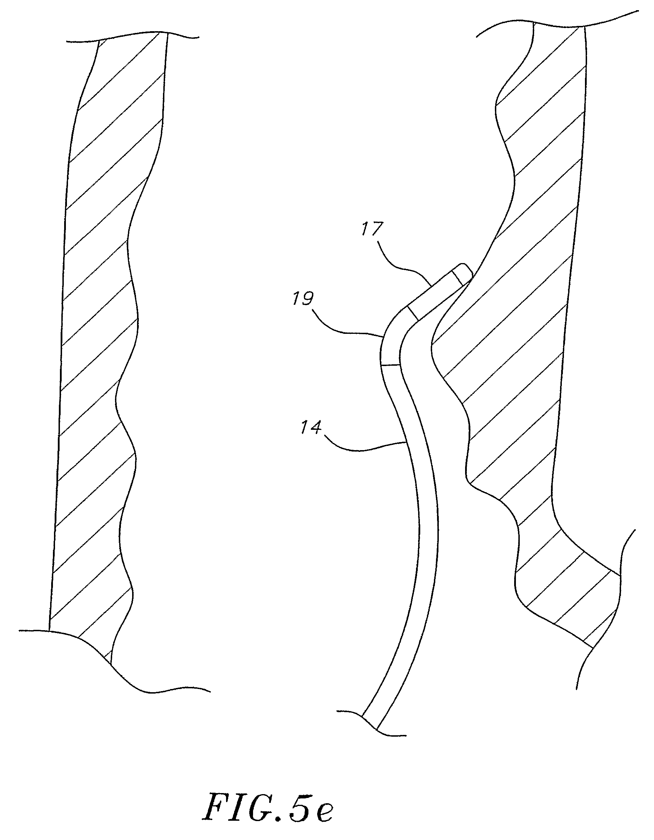

In FIG. 1, the catheter design is adapted for ablation of cavitary or tubular structures according to the method of introduction into the body as illustrated in FIGS. 5d and 5e although it is understood by one of ordinary skill in the art that the tip assembly may be adapted for mapping applications, as well.

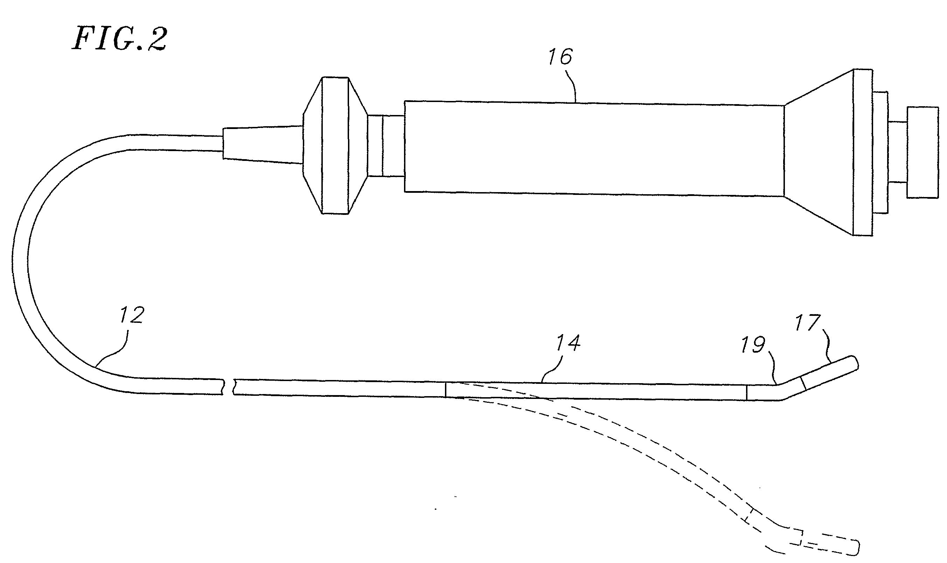

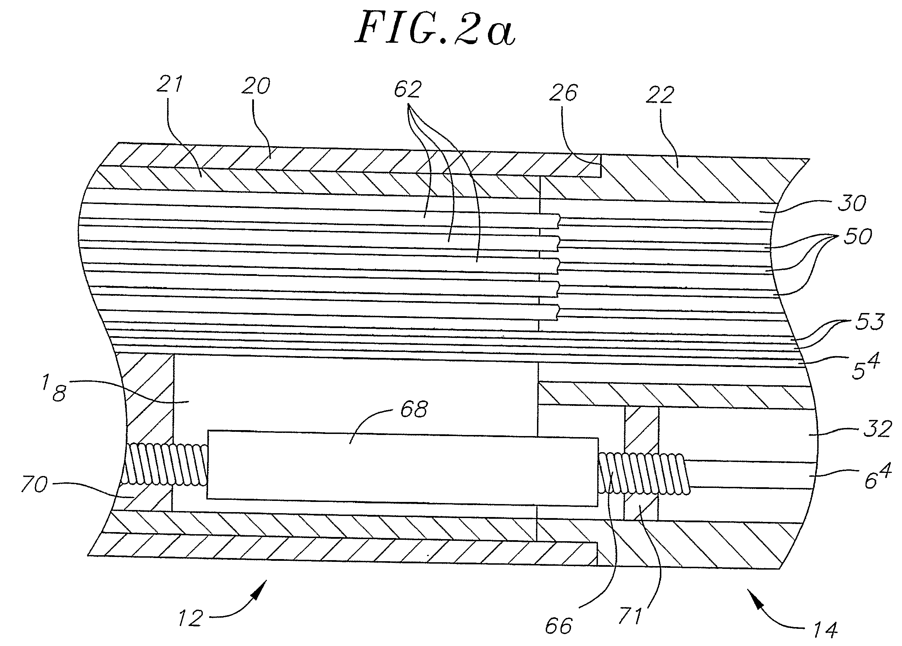

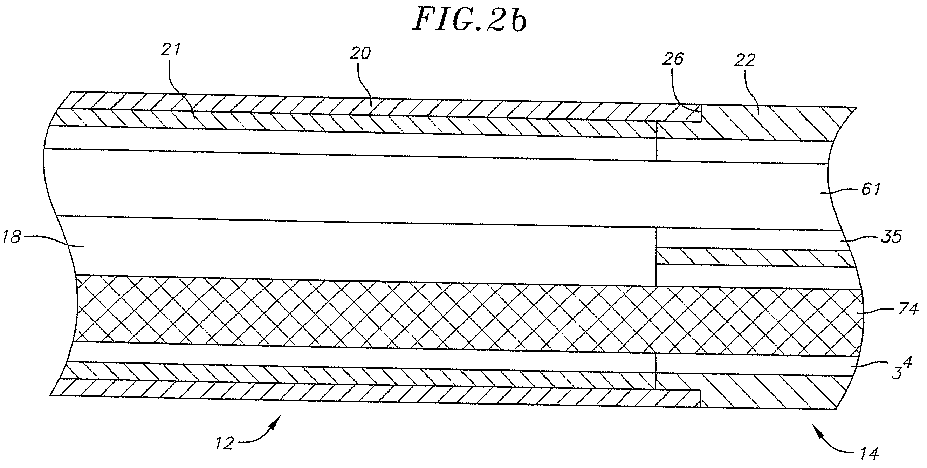

Referring to FIG. 2, the present disclosure also provides a catheter **10** having a tip assembly **17** for mapping and/or ablation at its distal end. The catheter comprises an elongated catheter body **12** having proximal and distal ends, a deflectable intermediate section **14** at the distal end of the catheter body **12,** and a control handle **16** at the proximal end of the catheter body. The tip assembly **17** is connected to the deflectable intermediate section **14** by a flexible section **19** which enables the tip assembly **17** to extend from the intermediate section **14** either in plane with or at a preset off-axis angle and/or off-plane angle.

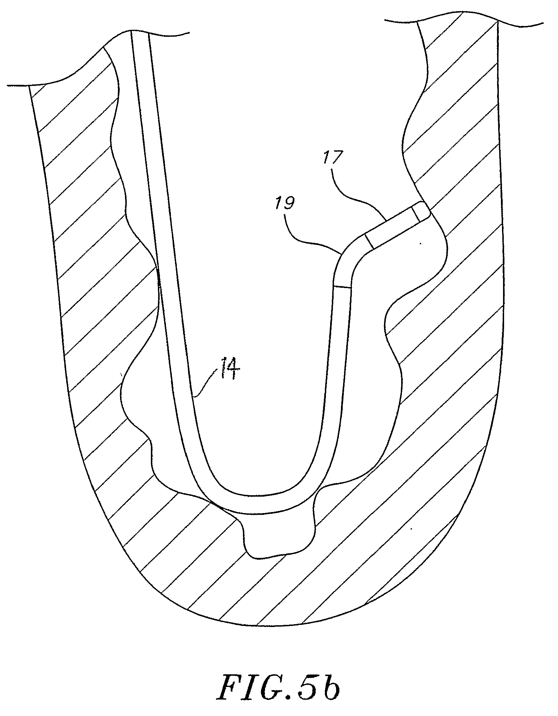

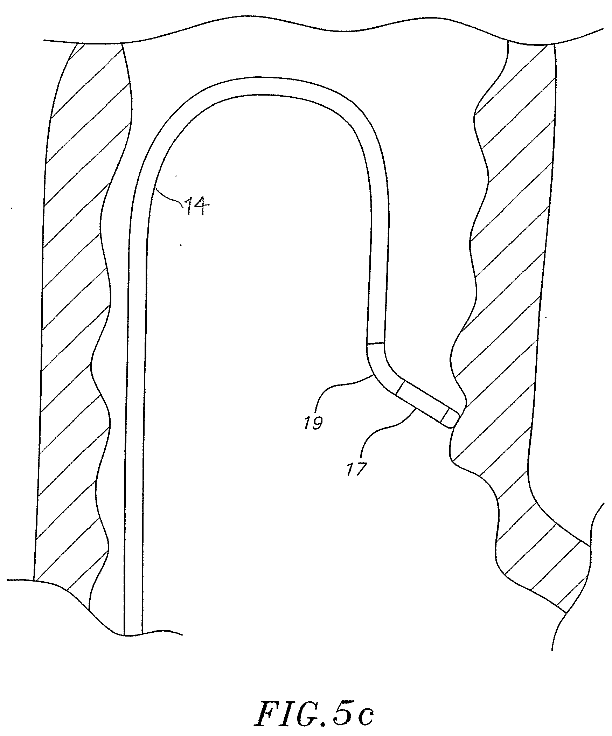

In FIGS. 5b and 5c, the tip assembly **17** is adapted for ablation of cavitary or tubular structures according to the method of introduction into the body, although it is understood by one of ordinary skill in the art that the tip assembly may be adapted for mapping applications, aswell.

With reference to **FIGs. 2a** and **2b****,** the catheter body **12** comprises an elongated tubular construction having a single, axial or central lumen **18.** The catheter body **12** is flexible, i.e., bendable, but substantially non-compressible along its length. The catheter body **12** can be of any suitable construction and made of any suitable material. A presently preferred construction comprises an outer wall **20** made of polyurethane or PEBAX. The outer wall **20** comprises an embedded braided mesh of stainless steel or the like to increase torsional stiffness of the catheter body **12** so that, when the control handle **16** is rotated, the intermediate section **14** of the catheter **10** is able to rotate in a corresponding manner.

The outer diameter of the catheter body **12** is not critical, but is preferably no more than about 9 french, more preferably about 7 french. Likewise, the thickness of the outer wall **20** is not critical, but is thin enough so that the central lumen **18** can accommodate a puller wire, one or more lead wires, and any other desired wires, cables or tubes. If desired, the inner surface of the outer wall **20** is lined with a stiffening tube **21** to provide improved torsional stability. A particularly preferred catheter **10** has an outer wall **20** with an outer diameter of from about 0.090 inches to about 0.094 inches and an inner diameter of from about 0.061 inches to about 0.065 inches.

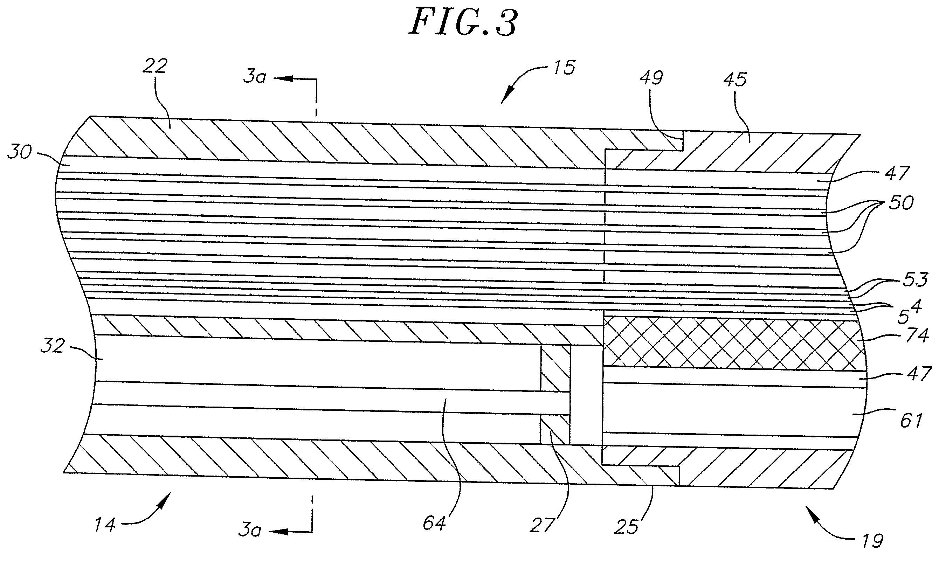

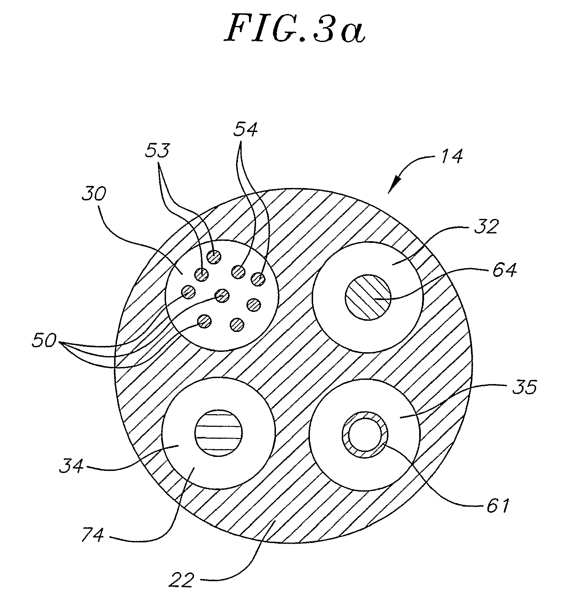

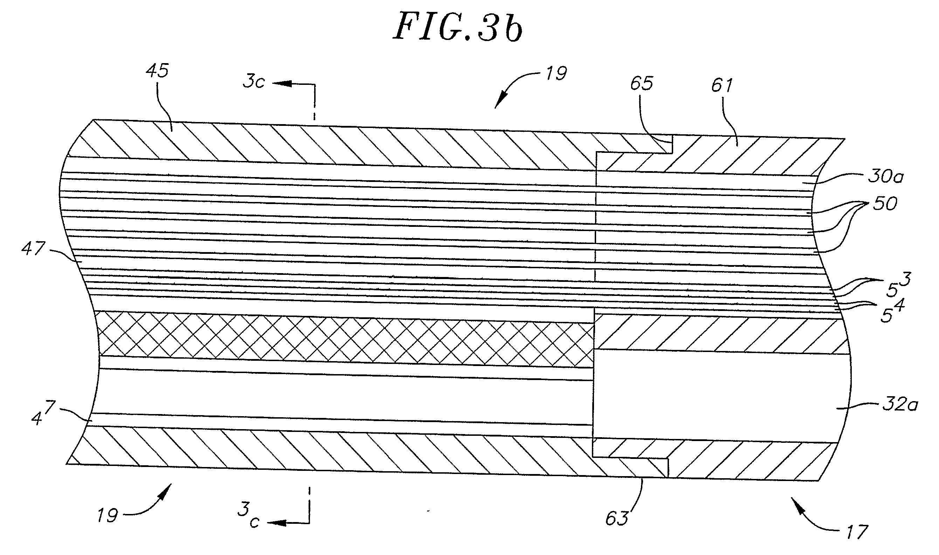

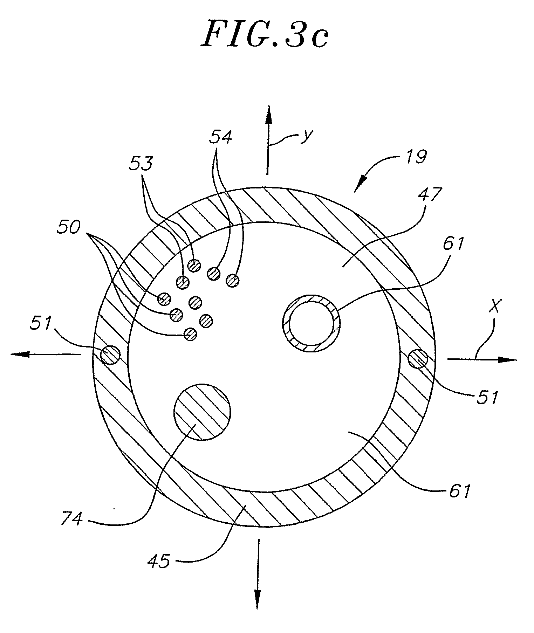

The intermediate section **14** comprises a short section of tubing **22** having multiple lumens, is shown in **FIG. 3a****.** In one example, a first lumen **30** carries one or more lead wires **50** and any other components (e.g., thermocouple wires **53** and **54** for monitoring tissue temperature) extending along the catheter **(****FIGS. 2a****, and** **3****).** A second lumen **32** carries a puller wire **64 (****FIG. 3****).** As also shown in **FIG. 2b****,** a third lumen **34** carries an electromagnetic sensor cable **74,** and a fourth lumen 35 carries an irrigation tube **61** for supplying fluid to the tip assembly **17.** The tubing **22** is made of a suitable non-toxic material that is preferably more flexible than the catheter body **12.** A presently preferred material for the tubing 22 is braided polyurethane, i.e., polyurethane with an embedded mesh of braided stainless steel or the like. The number of lumens or the size of each lumen is not critical, but is sufficient to house the lead wires, puller wire, electromagnetic sensor cable, thermal sensors and/or irrigation tube(s) depending on the circumstances.

The useful length of the catheter **10,** i.e., that portion that can be inserted into the body excluding the tip assembly **17,** can vary as desired. Preferably the useful length ranges from about 110cm to about 120cm. The length of the intermediate section **14** is a relatively small portion of the useful length, and preferably ranges from about 3.5c m to about 10cm, more preferably from about 5cm to about 6.5cm.

A preferred means for attaching the catheter body **12** and the intermediate section **14** is illustrated in **FIGs. 2a** and **2b****.** The proximal end of the intermediate section **14** comprises an outer circumferential notch **26** that receives the inner surface of the outer wall20 of the catheter body **12.** The intermediate section **14** and catheter body **12** are attached by glue or the like.

If desired, a spacer (not shown) can he located within the catheter body between the distal end of the stiffening tube **21** and the proximal end of the intermediate section **14.** The spacer provides a transition in flexibility at the junction of the catheter body **12** and intermediate section **14,** which allows the junction to bend smoothly without folding or kinking. A catheter having such a spacer is described in U.S. Patent No. 5,964,757..

As shown in **FIG. 2a****,** the puller wire **64** is provided for deflection of the intermediate section **14** (see **FIG. 1A**). The puller wire **64** extends through the catheter body **12. Its** proximal end is anchored to the control handle **16,** and its distal end is anchored to the distal end of the intermediate section **14** in the lumen **32** by any suitable means, for example, adhesives forming glue joint **27 (****FIG. 3****).** The puller wire **64** is made of any suitable metal, such as stainless steel or Nitinol, and is preferably coated with Teflon® or the like. The coating imparts lubricity to the puller wire **64.** The puller wire **64** preferably has a diameter ranging from about 0.006 to about 0.010 inch.

A compression coil 66 is situated within the catheter body **12** in surrounding relation to the puller wire **64,** as shown in **FIG. 2a****,** the compression coil **66** extends from the proximal end of the catheter body **12** to the proximal end of the intermediate section **14.** The compression coil 66 is made of any suitable metal, preferably stainless steel. The compression coil **66** is tightly wound on itself to provide flexibility, i.e., bending, but to resist compression. The inner diameter of the compression coil **66** is preferably slightly larger than the diameter of the puller wire **64.** The Teflon® coating on the puller wire **64** allows it to slide freely within the compression coil **66.** The outer surface of the compression coil **66** is covered by a flexible, non-conductive sheath **68,** e.g., made of polyimide tubing.

The compression coil **66** is anchored to the outer wall of the catheter body **12** by proximal glue joint **70** and at its distal end to the intermediate section **14** by distal glue joint **71.** Both glue joints **70** and 71 preferably comprise polyurethane glue or the like. The glue may be applied by means of a syringe or the like through a hole made between the outer surface of the catheter body **12** and the central lumen **18.** Such a hole may be formed, for example, by a needle or the like that punctures the outer wall **20** of the catheter body **12** which is heated sufficiently to form a permanent hole. The glue is then introduced through the hole to the outer surface of the compression coil **66** and wicks around the outer circumference to form a glue joint about the entire circumference of the compression coil.

Longitudinal movement of the puller wire **64** relative to the catheter body **12,** which results in deflection of the intermediate section **14,** is accomplished by suitable manipulation of the control handle **16.** Examples of suitable control handles for use in the present invention are disclosed in U.S. Patent Nos. Re 34,502 and 5,897,529.

As mentioned, deflection of the intermediate section **14** by longitudinal movement of the puller wire **64** allows the intermediate section **14** to generally approximate and conform to the convex curvature of the isthmus. As such, the deflected intermediate section **14** can sit on the isthmus and transmit the motion of the heart during systole, diastole and respiration to the entire catheter. The distal tip of the catheter is thus both stable and moves in synchrony with the heart. This allows the tip assembly of the catheter to conform to irregularities without undue pressure reducing the risk of any of the following: a) direct mechanical perforation because the flexible section readily flexes so as to reduce the maximal tip pressure that can be applied by the proximal portion of the catheter, b) perforation due to steam pop, as the flexible section allows the tip assembly to be displaced off the surface allowing the steam to exit into the right atrium rather than the tip pressure forcing the steam into the myocardium and out into the pericardial space; c) impedance rise, excess temperature, thrombus and char formation, as the maximum tip pressure is limited by the flexible section reducing the likelihood of the tip assembly being buried in the tissue, reducing cooling by the circulating blood.

For example, the tip assembly **17** is attached to the intermediate section **14** by the pre-shaped flexible section **19.** As shown in **FIG. 4****,** the flexible section **19** supports the tip assembly **17** at an in-plane off-axis angle from the distal end of the intermediate section **14.** Using an angle **θ** to define the off-axis angle, the angle **θ** may range between about 0 degrees to about 90 degrees, preferably between about 10 degrees to 60 degrees, and more preferably about 30 degrees. With the tip assembly **17** in plane but canted off axis in the direction of deflection of the intermediate section **14,** the angle **θ** effectively increases the deflection angle to enable the tip assembly **17** to reach further around the isthmus and contact the tissue surface. The flexibility of the section **19** allows the angle **θ** to be varied from the initially set angle to zero degree (or on-axis position) with minimal force applied to the tip assembly **17** through contact with the tissue.

The flexible section **19** is constructed with sufficient shape memory and/or sufficient flexibility and elasticity so that the tip assembly **17** can temporarily assume a different (greater or lesser) angle **θ** as needed for the tip assembly to pivot at its proximal end. The flexible section **19** can be sufficiently soft to allow the tip assembly **17** to be displaced from its preset off-axis angle **θ** to an on-axis angle where **θ** is about zero, and sufficiently elastic to return (or at least bias the return of) the tip assembly **17** to its preset off-axis angle **θ** thereafter, whether the displacement was caused by a formation **37** in the tissue surface, the tip assembly being caught or buried in the surrounding tissue, or a "steam pop" where a build up of pressure dislodges the tip assembly from tissue contact. To that end, the flexible section **19** has a relatively high flexural modulus measuring on a Durometer scale no greater than about 25D to 35D and/or no greater than about 1/2 to 1/4 of the Durometer measurement of the intermediate section **14.** The flexible section **19** acts as a "shock absorber" when the tip assembly is jarred or otherwise displaced from its preset position. The flexible section **19** enables the tip assembly **17** to pivot away from the recess **37** independently of the intermediate section **14** so that the tip assembly can remain in contact with the tissue. Referring to Fig. 4, as the catheter **10** is advanced, withdrawn or otherwise maneuvered around the isthmus, the tip assembly **17** can move from its preset angle **0** (solid lines) to a displaced position at angle **0'** (broken lines) without significantly displacing the intermediate section **14** whether or not deflected.

As understood by one of ordinary skill in the art, the shape memory of the material **45** of the flexible section **19** also allows the catheter to be advanced atraumatically in the patient's body in a generally straight configuration through a vein or artery and yet be able to assume its preformed shape when it reaches the heart.

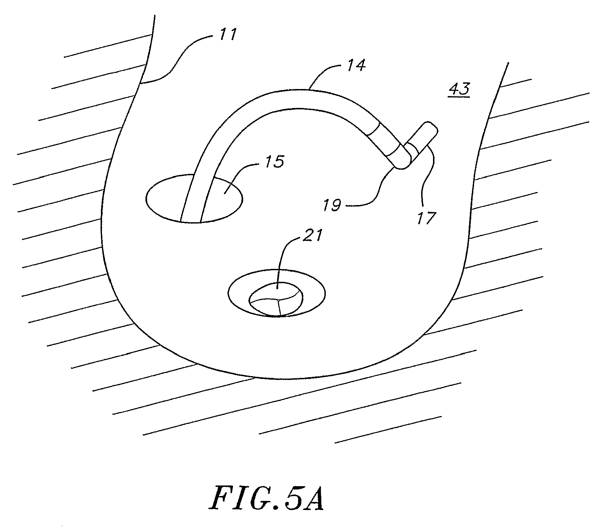

Referring to **FIGS. 5** and **5A****,** in accordance with the present invention the highly flexible section **19** is configured to support the tip assembly **17** off-plane from the intermediate section **14** at a variety of radial angles. As shown in **FIG. 5A****,** the catheter **10** is adapted to map and/or ablate another region in the right atrium with a generally convex contour, such as the Bundle of His region **43** (or "His region" hereinafter), although the His region may pose a further challenge as the region is also slightly canted anteriorly from the inferior vena cava **15.**

The His region **43** is accessible to the catheter **10** despite the catheter's entry to the atrium from the inferior vena cava **15** and the catheter's forward approach to the His region. As with the foregoing embodiment, the intermediate section **14** is deflected so the tip assembly **17** can reach the His region. Where the deflected intermediate section **14** can approximate and assume a convex curvature near the His region, motion of the heart is transferred to catheter to stabilize the catheter. In accordance with a feature of the present invention, contact between the tip assembly **17** and tissue surface of the His region **43** is enabled by an off-plane extension of the tip assembly **17** (which may or may not also extend at an off-axis angle from the intermediate section **14**). The highly flexible section **19** between the tip assembly **17** and the intermediate section **14** allows the tip assembly to maintain contact with the His tissue surface despite the uneven and nonuniform surface of the His region which has recesses and protrusions that are encountered by the tip assembly **17** as it is dragged along to map and/or ablate the His region.

Referring to **Fig. 5** (a top view of the tip assembly **17** and intermediate section **14),** using angle γ to define the radial angle from plane of deflection 33 of the intermediate section **14,** the angle **γ** may range between about 0 to 180 degrees, preferably about 20 to 90 degrees, and more preferably about 45 degrees as shown in the embodiment of **Fig. 5** (compared with the embodiment of **FIG. 4a** where the angle γ is about zero degrees).

As illustrated in **Fig. 5A****,** where the catheter enters the right atrium from the inferior vena cava, the off-plane radial angle γ of the tip assembly **17** extending from the deflected intermediate section **14** allows the tip assembly to reach in an angle generally lateral of the deflection direction. As such, the His region is readily accessed by the tip assembly **17** for mapping and/or ablation.

It is understood by one of ordinary skill in the art that the off axis angle **0** and the off-plane angle γ may be preset independently of one another. That is, the catheter **10** of the present invention may have the tip assembly **17** extend from the intermediate section **14** at any combination of the angle **0** and the angle γ in accordance with their respective ranges set forth above, as desired or appropriate. In one embodiment of the catheter **10** for use in ablating and/or mapping the His region, the angle **0** is about 20 degrees and the angle γ is about 90 degrees.

As mentioned, the flexible section **19** allows the tip assembly to be displaced without displacing the intermediate section 14. In one embodiment, the tip assembly **17** can be displaced from its off-plane angle under a force or weight of merely about 0.25 to about 2.0oz, and more preferably about 1.0 ounce. As such, the flexible section **19** provides sufficient flexibility to reduce the risk of injury that can result from the tip assembly **17** inadvertently perforating tissue or being buried in the tissue and overheating. As understood by one of ordinary skill in the art, the force required to displace or capable of displacing the tip assembly from the preset angle(s) also depends on the point of application of the force to the tip assembly, as well as the length of the tip assembly.

The flexible section **19** comprises a short section of material **45** (e.g., tubing) with a central lumen **47** through which the lead wire(s) **50,** thermocouple wires **53** and **54,** sensor cable **74** and irrigation tube **61** extend distally and connect to the tip assembly **17.** A junction **25** of the intermediate section **14** and the flexible section **19** is shown in **FIG. 3****.** The proximal end of the material **45** of the tip assembly **17** comprises an outer circumferential notch **49** that receives the inner surface of the tubing **22** of the intermediate section **14.** The intermediate section **14** and the flexible section **19** are attached by glue or the like. The flexible section can be made of polyurethane, PEBAX, silicone or combinations thereof and is preformed (used generally interchangeably with "preshaped" herein) with shape memory by placing the tubing **45** in a delrin mold and heating the mold at about 100°C for about 30 minutes. Alternatively, the tip assembly and the flexible section may be formed as a single unit with the flexibility of the tip assembly or flexible section determined by the incorporated sensors, wires and electrodes. The length of the flexible section **19** can vary as desired and can range between about 0.1cm and 2.0cm, preferably between about 0.2cm and 1.0cm, and more preferably about 5.0cm.

Moreover, where desirable or appropriate, lateral stability can be provided in the tip assembly **17** with the use of struts or ribbons **51** provided in walls of the material **45** of the flexible section **19,** as shown in **FIG. 3c****,** or elsewhere on or in the tubing as desirable. A pair of struts **51** can be aligned along any diameter of the material **45** to stabilize the tip assembly. In the embodiment of **Fig. 3c****,** the struts minimize lateral movement along direction **X** but still allow displacement along direction **Y.**

Recognizing that atria and isthmuses can come in different shapes and sizes, the intermediate section 14 may have a length ranging between about 1.0cm and 20cm, preferably between about 4.0c m and 16cm, and more preferably between about 7.0cm and 12cm. The intermediate section 14 may assume a "J" curve when deflected for flutter treatment and procedures and a "D" curve for HIS treatment and procedures. However, it is understood that the intermediate section and its deflection curvature may assume a variety of sizes and shapes as desirable or appropriate for the intended region of ablation or mapping.

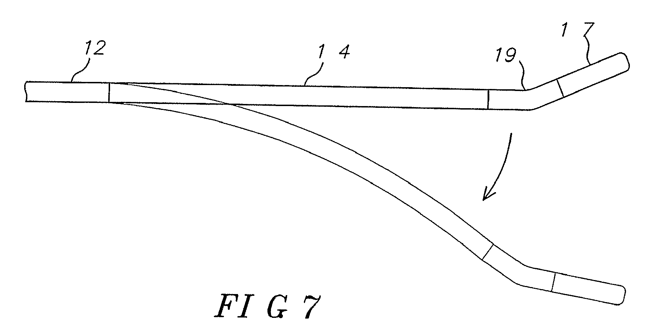

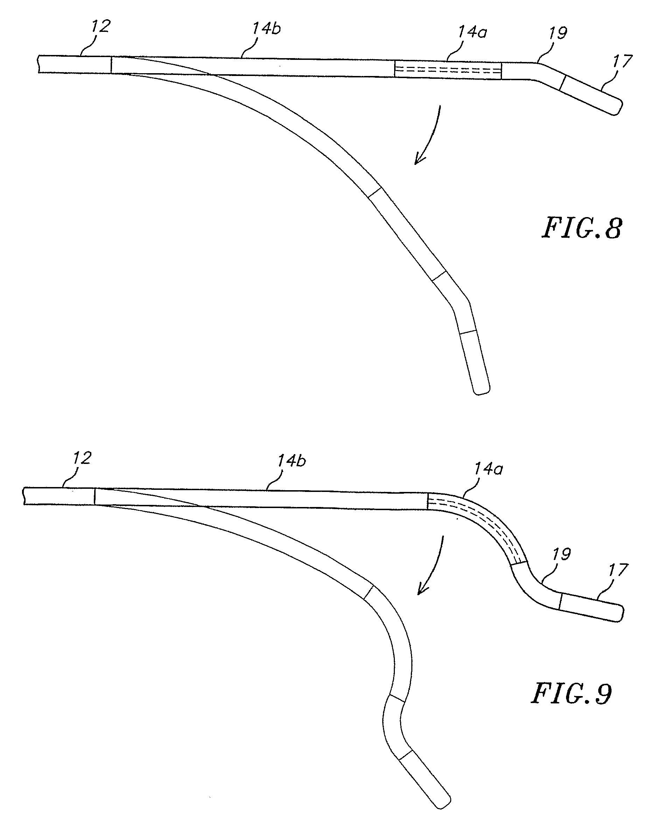

In addition, as shown in **FIGS. 7****,** **8 and 9****,** it is understood by one of ordinary skill in the art that the flexible section **19** may flexibly extend the tip assembly **17** in a direction generally opposite to the direction of deflection of the intermediate section **14 (****Fig. 7****),** or that the intermediate section may be divided into distal and proximal sections **14a and 14b** with the proximal intermediate section **14b** deflectable and the distal intermediate section **14a** with shape-memory configured generally straight **(****FIG. 8****)** or with a curve **(****FIG. 9****),** as desirable or appropriate for the intended region of ablation or mapping.

In illustrated example, the tip assembly **17** comprises a short section of material tubing **61** (e.g., tubing) (**FIGs**. **3b****,** **5** **A and 5 b)** comprising four lumens **30a, 32a, 34a** and **35A,** generally corresponding to and aligned with the four lumens **30, 32, 34** and **35** respectively, of the intermediate section **14.** The length of the tip assembly **17** can be varied as desired, but preferably ranges between about 8mm to about 15mm, and more preferably is about 10mm. Ajunction **63** of the flexible section **19** and the tip assembly **17** is shown in **FIG. 3B****.** The proximal end of the material **61** of the tip assembly **17** comprises an outer circumferential notch **65** that receives the inner surface of the tubing **45** of the flexible section **19.** The flexible section **19** and tip assembly **17** are attached by glue or the like.

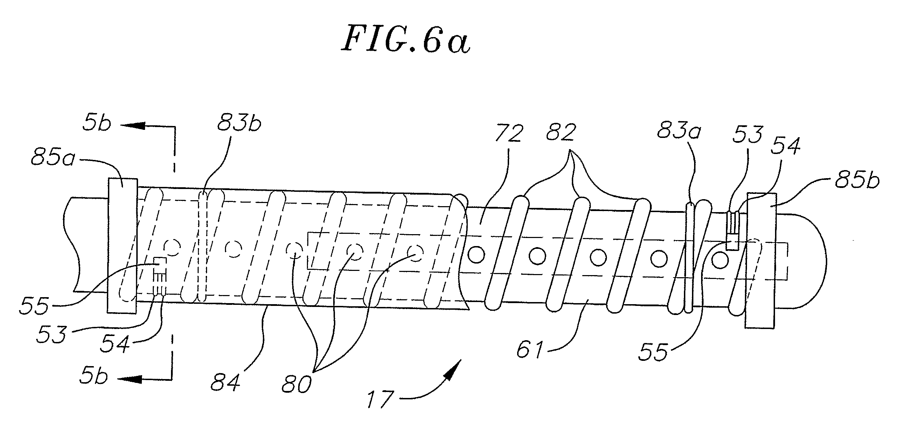

**FIG. 6a** illustrates the tip assembly **17** configured as an ablation assembly. A coil electrode **82** is coiled around the length of the ablation assembly **17.** The longitudinal span of the coil electrode **82** may be made of any suitable metal, preferably platinum/iridium and ranges in length from about 6 to about 10mm, preferably about 8mm to generally match the length of the ablation assembly **17.**

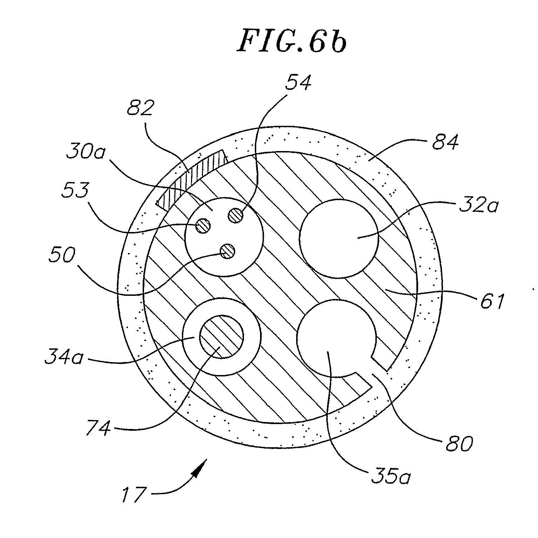

In the disclosed embodiment, the ablation assembly **17** is irrigated and comprises a plurality of irrigation ports **80** disposed along most of the length of the ablation assembly **17** through which fluid can pass to the outer surface of the ablation assembly to cool the ablation site. The coil and the irrigation ports **80** are arranged so that an irrigation port lies between each wind of the coil electrode **82.** The irrigation ports may comprise round holes formed on the surface of the tubing **61** on the side of the ablation assembly **17** in communication with the fourth lumen **35A** which is supplied fluid by the irrigation tube **61** whose distal end is slightly proximal of the most proximal irrigation port. Any number of irrigation ports **80** may be used. In the illustrated example, the tubing **61** of the ablation assembly **17** is configured with about 10 irrigation ports **80.** The circumference of each round hole can measure about 20/1000 inch. As shown in **FIGS. 6a** **and** **6b****,** a porous protective covering **84,** of, for example, expanded polytetrafluoroethylene (EPTFE), is disposed over the tubing 61in surrounding relation to and covering the coil electrode **82** and irrigation ports **80**

A tip electrode lead wire **50 (****FIG. 6b****)** connects the coil electrode **82** to a suitable source of ablation energy (not shown), preferably radio frequency (RF) energy. The distal end of the lead wire **50** is attached to the proximal end of the coil electrode 82. The proximal end of the lead wire **50** is electrically connected to the source of ablation energy as is known in the art. The lead wire **50** extends through the first lumen **30a** of the ablation assembly **17,** the central lumen **47** of the flexible section **19,** the first lumen **30** of the intermediate section **14,** the central lumen **18** of the catheter body **12,** and the control handle **16,** and terminates at its proximal end in a connector (not shown).

As shown in **FIG. 6a****,** if desired, mapping and/or ablation ring electrodes **83a** and **83h** maybe mounted on the ablation assembly **17.** Additional ring electrodes may be contained within the ablation assembly or the intermediate section depending on spacing or the application of the catheter. The ring electrodes **83a** and **83h** can be mounted over the coil electrode **82** and underneath the porous covering **84.** In the illustrated example, the first ring electrode **83a** is positioned in between the two distal most irrigation ports **80.** The second ring electrode **83h** is positioned in between the two proximal most irrigation ports **80.** The ring electrodes **83a** and **83b** are mounted to the coil electrode **82** by any suitable means, for example by welding, soldering or the like. As such, the ring electrodes **83a** and **83b** are electrically connected to the coil electrode **82** and its associated lead wire for ablation purposes. The ring electrodes **83a** and **83b** serve in part to hold the coil electrode **82 in** place on the tubing 61 of the ablation assembly. The ring electrodes **83a and 83b** also serve to flatten the coil electrode **82** on the surface of the tubing **61,** thereby preventing any rough edges of the coil electrode **82** from cutting into the porous covering **84.**

Any conventional temperature sensors, e.g. thermocouples or thermistors, may be used. In **FIGs. 2a****,** **3** **and** **6a****,** the temperature sensors comprise two thermocouples formed by two enameled wire pairs. One wire of each wire pair is a copper wire **53,** e.g., a number "40" copper wire. The other wire of each wire pair is a constantan wire **54.** The wires **53** and **54** of each wire pair are electrically isolated from each other except at their distal ends where they are twisted together, covered with a short piece of plastic tubing **55 (****FIG. 6a****),** e.g., polyimide, and covered with epoxy. The wires **53** and **54** of each wire pair extend out a hole in the side wall of the tubing **61** and are anchored to the outer surface of tubing **61.** The hole in the side wall of the distal region is sealed by a plug. Any suitable seal may be used, for example glue or the like. Each plastic tubing **55** is mounted on the outer surface of the tubing **61** by polyurethane glue or the like. One of the two thermocouples is anchored immediately distal the distal most irrigation port **80,** as shown in **FIG. 6a****.** The second of the two thermocouples is anchored immediately proximal the proximal most irrigation port **80.** The wires **53** and **54** extend through the first lumen **30** in the ablation assembly **17** and intermediate section **14,** through the central lumen **18** of the catheter body **12** and out through the control handle **16** to a connector (not shown) connectable to a temperature monitor (not shown).

Additional electrodes may be incorporated depending on the application electrode width and spacing, as well as the preferences of the operator of the catheter. If desired, one or more mapping and/or ablation ring electrodes can be mounted on the tubing **45** of the flexible section **19** and tubing **61** of the ablation assembly **17,** as shown in **FIGs. 5** **and** **6a****.** These ring electrodes might be desirable, for example, for mapping the region to be ablated before ablation begins or after ablation to assure that the lesions blocked the electrical activity as desired. A ring electrode **85A** can be mounted on the proximal end of the tubing **61** of the ablation assembly **17** over the porous covering **84** so that the proximal end of the porous covering **84** can be tucked underneath the ring electrode **85A** to lock the proximal position of the porous covering **84.** Also, a second ring electrode **85b** can be mounted on the distal end of the tubing 61 so that the distal end of the porous covering **84** can be tucked underneath the ring electrode **85b** to lock the distal position of the porous covering **84.**

In other examples, the tip assembly **17** whether adapted for mapping or ablation may be constructed with or without irrigation, with or without temperature sensors, using suitable ring electrodes for sensing and/or ablation, as understood by one of ordinary skill in the art. The relationship between the tip assembly and the flexible sectionremains generally as described herein.

In addition, as better shown in **FIGs.** 4 **and 4A****,** two additional ring electrodes **86a** and **86b** for mapping are mounted on the flexible section **19.** The first ring electrode **86a** is positioned approximately 5 mm proximal the proximal locking ring electrode **85A** and is used to confirm the position of the ablation assembly in the atrium. The second ring electrode **86b** is positioned approximately 2.5 mm proximal the first ring electrode **86a** and is also used to confirm the position of the ablation assembly in the atrium. As understood by one of ordinary skill in the art, the mapping electrodes may be mounted at different locations on the ablation assembly **17,** flexible section **19** and/or intermediate section **14** as desired.

In **FIG. 3****,** each ring electrode **85A, 85b, 86a, 86b and 86c** is connected to a corresponding lead wire **50.** The distal end of each lead wire **50** is attached to the corresponding ring electrode. The proximal end of each lead wire **50** is electrically connected to a suitable monitoring device for monitoring electrical activity. Each lead wire **50** extends through the first lumen **30a** of the ablation assembly **17,** the central lumen **47 of** the tubing **45,** the first lumen **30** of the intermediate section **14,** the central lumen **18** of the catheter body **12,** and the control handle **16,** and terminates at 'its proximal end in a connector (not shown).

As shown in **FIG. 2a****,** the portion of each lead wire **50** extending through the control handle 16, the central lumen **18** of the catheter body **12,** and at least the proximal section of the intermediate section **14** is enclosed within a protective sheath **62** to prevent contact with other lead wires or other components of the catheter. The protective sheath **62** can be made of any suitable material, preferably polyimide. The protective sheath **62** is anchored at its distal end to the proximal end of the intermediate section **14** by gluing it in the first lumen **30** with polyurethane glue or the like. As would be recognized by one skilled in the art, the protective sheath **62** can be eliminated if desired.

As shown in **FIG. 6a****,** an electromagnetic navigation sensor **72** may be contained within the ablation assembly **17.** The electromagnetic sensor **72 is** preferably situated at the distal tip of the ablation assembly **17** and is approximately 5mm long. The electromagnetic sensor **72** is positioned in the third lumen **34a** of the ablation assembly **17.** The electromagnetic sensor **72** is mounted to the tubing **61** of the ablation assembly **17** by any suitable means, e.g. by polyurethane glue or the like.

The electromagnetic sensor **72** is connected to an electromagnetic sensor cable **74,** which extends through the third lumen **34a** in the ablation assembly **17,** the central lumen **47** of the flexible section **19,** the third lumen **34** of the intermediate section **14,** through the catheter body **12,** and out through the control handle **16.** The electromagnetic sensor cable **74** comprises multiple wires encased within a plastic covered sheath. In the control handle **16,** the sensor cable **74** is connected to a circuit board (not shown). The circuit board amplifies the signal received from the electromagnetic sensor **72** and transmits it to a computer in a form understandable by the computer. Because the catheter is designed for a single use only, the circuit board may contain an EPROM chip which shuts down the circuit board approximately 24 hours after the catheter has been used. This prevents the catheter, or at least the electromagnetic sensor from being used twice.

Suitable electromagnetic sensors for use with the present invention are described, for example, in U.S. Patent Nos. 5,558,091,5,443,489, 5,480,422, 5,546,951, and 5,391,199.

A preferred electromagnetic sensor **72** has a length of from about 6 mm to about 7 mm, preferably about 5 mm, and a diameter of about 1.3 mm.

In **FIG. 3a****,** the irrigation tube **61** may be made of any suitable material, and is preferably made of polyimide tubing. A preferred irrigation tube has an outer diameter of from about 0.032 inch to about 0.036 inch, and an inner diameter of from about 0.028 inch to about 0.032 inch. The irrigation tube **61** extends through the central lumen **18** of the catheter body **12 (****FIG. 2b****),** the fourth lumen **35** of the intermediate section **14,** the central lumen **47** of the flexible section **19,** and the fourth lumen **35A** of the ablation assembly **17 (****FIG. 3a****),** and terminates slight proximal of the most proximal irrigation port **80** in the ablation assembly **17.** The proximal end of the irrigation tube **61** extends through the control handle **16** and terminates in a luer hub or the like (not shown). Fluid is introduced into the irrigation tube **61** through the luer hub. The fluid, e.g. saline, is then introduced to the fourth lumen **35A** of the ablation assembly **17** by the irrigation tube **61** and passes to the outer surface of the tubing **61** through the irrigation ports **80** (FIG. 5A). The fluid is then dispersed over generally the entire surface of the ablation assembly 17 by the porous covering **84.** This irrigation enables creation of deeper lesions.

In use, the catheter **10** is inserted into the patient through a suitable guiding sheath whose distal end is positioned at a desired mapping or ablating location. An example of a suitable guiding sheath for use in connection with the present invention is the Preface™ Braided Guiding Sheath, commercially available from Biosense Webster, Inc. (Diamond Bar, California). The distal end of the sheath is guided into one of the atria. A catheter in accordance with the present invention is fed through the guiding sheath until its distal end extends out of the distal end of the guiding sheath. As the catheter **10** is fed through the guiding sheath, the tip assembly **17,** the flexible section **19** and the intermediate section **14** are generally straightened to fit through the sheath. Once the distal end of the catheter is positioned at the desired mapping or ablating location, the guiding sheath is pulled proximally, allowing the deflectable intermediate section **14,** the flexible section **19** and the tip assembly **17** to extend outside the sheath, and return to their original preformed shapes with the tip assembly **17** extending from the intermediate section **14** at a predetermined off-axis angle **θ** and/or off-plane angle γ.

In one embodiment, where the catheter is advanced into the right atrium, the intermediate section **14** is deflected to approximate the generally convex curvature of the cava-tricuspid isthmus or the His region where the intermediate section 14 can rest on the tissue and is stabilized and in synch with the motion of the heart.

With the intermediate section **14** deflected, the tip assembly **17** makes contact with tissue in the region by means of the preset off-plane angle(s) provided by the flexible section **19.** To create generally focal lesions during ablation, the ablation assembly is positioned and the flexible section **19** allows the ablation assembly to be readily displaced from contact with the tissue before damage can occur from perforation, steam build-up and the like. For continuous lesions during ablation, the tip assembly **17** is dragged along the tissue surface. As the ablation assembly encounters uneven formation such as a projection or recess in the tissue surface, the flexible section **19** flexes as the ablation assembly **17** pivots from the preset angle(s) to absorb the movement without affecting the intermediate section **14.** The catheter body may also be rotated to form a linear line of block at the His region. Because the off-plane angle allows the ablation assembly to reach tissue lateral of the plane of deflection, rotation of the ablation assembly (e.g., by rotation of the catheter body and/or the control handle) can create a generally linear ablation line.

Regardless of the ablation lesion desired, the tip assembly **17** maintains continuous contact with the tissue for improved lesions. In the embodiment of the catheter for mapping applications, similar manipulations of the catheter and the control handle enable the mapping electrodes **85A, 85b, 86a, 86b** and **86c** to map in a linear or circumferential pattern.

The preceding description has been presented with reference to presently preferred embodiments of the invention. Workers skilled in the art and technology to which this invention pertains will appreciate that the Figures are not necessarily to scale and alterations and changes in the described structure may be practiced without meaningfully departing from the scope of the invention as defined by the appended claims.

## Claims

1. A catheter comprising:
an elongated flexible tubular catheter body (12) having proximal and distal ends;
an intermediate section (14) attached to the distal end of the catheter body, wherein the intermediate section is deflectable in a first plane to approximate a generally convex region of the heart;
a tip assembly (17); and
a flexible section (19) connecting the tip assembly to the intermediate section at a preset off-plane angle such that the tip assembly extends in a second plane that is different from the first plane, the flexible section adapted to permit displacement of the tip assembly from the preset angle without displacing the intermediate section;
a puller wire (64) having proximal and distal ends extending through the catheter body, the distal end of the puller wire being fixedly attached within a distal end of the intermediate section;
a control handle (16) connected to the proximal ends of the catheter body and puller wire for moving the puller wire longitudinally relative to the catheter body, whereby longitudinal movement of the puller wire relative to the catheter body results in deflection of the intermediate section;
wherein the intermediate section is more flexible than the catheter body and the flexible section is more flexible than the intermediate section; and
the durometer measurement of the flexible section is no greater than approximately ½ to ½ of the durometer measurement of the intermediate section.

2. The catheter of claim 1, wherein the intermediate section comprises a distal section (14a) and a proximal section (14b), the distal section having shape-memory.

3. The catheter of claim 2, wherein the distal section (14a) is curved.

4. The catheter of claim 2, wherein the proximal portion (14b) is deflectable.

5. The catheter of claim 1, wherein the flexible section connects the tip assembly (17) to the intermediate section (14) at a preset off-axis angle.

6. The catheter of claim 1, wherein the flexible section comprises at least one support structure (51) to minimize movement of the tip assembly (17) in a selected direction.

7. The catheter of claim 5, wherein the off-axis angle is in a direction of deflection of the intermediate section (14).

8. The catheter of claim 5, wherein the off-axis angle ranges between about 2 and 180 degrees generally in a direction of deflection of the intermediate section (14).

9. The catheter of claim 5, wherein the off-axis angle ranges between about 2 and 180 degrees generally opposite to a direction of deflection of the intermediate section (14).

10. The catheter of claim 1, wherein the off-plane angle ranges between about 2 and 180 degrees generally in a direction of deflection of the intermediate section (14).

11. The catheter of claim 1, wherein the off-plane angle ranges between about 2 and 180 degrees generally opposite to a direction of deflection of the intermediate section (14).

12. The catheter of claim 5, wherein the displacement of the tip assembly (17) is between the preset off-axis position and an on-axis position.

13. The catheter of claim 1, wherein the tip assembly (17) is configured for mapping.

14. The catheter of claim 1, wherein the tip assembly (17) is configured for ablation..

15. The catheter of claim 1, adapted to maintain the tip assembly (17) in contact with the tissue during movement of the catheter without affecting the intermediate section (14).

16. A catheter of claim 15, wherein a deflected intermediate section (14) generally conforms to a cavo-tricuspid isthmus region in the right atrium, a His region in the right atrium, a region of a right atrium, a region of a left atrium or a superior vena cava.

17. A catheter of claim 15, wherein contact between the intermediate section (14) and the generally convex region synchronizes the intermediate section to heart motion during systole, diastole or respiration.

18. A catheter of claim 15, wherein contact between the intermediate section (14) and the generally convex region generally stabilizes the tip assembly (17).

19. A catheter of claim 15, wherein the tip assembly includes an ablation electrode.

20. A catheter of claim 15, wherein the tip assembly includes a mapping electrode.

21. The catheter of claim 1, wherein the tip assembly (17) comprises an ablation assembly.

22. The catheter of claim 21, wherein the ablation assembly comprises:
a plurality of irrigation ports (80);
a coil electrode (82); and
a porous covering (84) in surrounding relation to the coil electrode and irrigation ports.

23. The catheter of claim 21, wherein the coil electrode (82) has a length ranging from about 8 mm to about 15 mm.

24. The catheter of claim 23, wherein the coil electrode (82) has a length of about 10mm.

25. The catheter of claim 22, wherein the porous covering (84) comprises expanded polyletrafluoroelhylene.

26. The catheter of claim 21, further comprising a temperature sensor mounted in the ablation assembly.

27. The catheter of claim 22, further comprising:
a proximal locking ring electrode (85A) mounted on a proximal region of the ablation assembly over the coil electrode and the porous covering; and
a distal locking ring electrode mounted on a distal region of the ablation assembly over the coil electrode and the porous covering.

28. The catheter of claim 21, further comprising one or more ring electrodes proximal of the ablation assembly.

29. The catheter of claim 21, further comprising an electromagnetic sensor (72) mounted in the ablation assembly.

30. The catheter of claim 21, wherein the intermediate section (14) further comprises a support member comprising a material having shape-memory to provide the performed curve.

31. The catheter of claim 21, further comprising an irrigation tube (61) extending into the ablation assembly.

## Patentansprüche

1. Katheter, umfassend:
einen langgestreckten flexiblen schlauchförmigen Kathetherkörper (12) mit proximalen und distalen Enden;
einen Zwischenabschnitt (14), der an dem distalen Ende des Katheterkörpers befestigt ist, wobei der Zwischenabschnitt in einer ersten Ebene ablenkbar ist, um sich einer allgemein konvexen Herzregion anzunähern;
eine Spitzenanordnung (17); und
einen flexiblen Abschnitt (19), der die Spitzenanordnung mit dem Zwischenabschnitt in einem voreingestellten Winkel außerhalb der Ebene verbindet, so dass sich die Spitzenanordnung in eine zweite Ebene erstreckt, die sich von der ersten Ebene unterscheidet, wobei der flexible Abschnitt ausgelegt ist, eine Verschiebung der Spitzenanordnung von dem voreingestellten Winkel ohne Verschiebung des Zwischenabschnitts zu gestatten;
einen Zugdraht (64) mit proximalen und distalen Enden, die sich durch den Katheterkörper erstrecken, wobei das distale Ende des Zugdrahts fest in einem distalen Ende des Zwischenabschnitts befestigt ist;
einen Steuergriff (16), der mit den proximalen Enden des Katheterkörpers und des Zugdrahts verbunden ist, um den Zugdraht längs relativ zum Katheterkörper zu bewegen, wobei die Längsbewegung des Zugdrahts relativ zum Katheterkörper zur Ablenkung des Zwischenabschnitts führt;
wobei der Zwischenabschnitt flexibler als der Katheterkörper ist und der flexible Abschnitt flexibler als der Zwischenabschnitt ist; und
die Durometer-Messung des flexiblen Abschnitts nicht größer als ungefähr ½ bis ¼ der Durometer-Messung des Zwischenabschnitts ist.

2. Katheter nach Anspruch 1, wobei der Zwischenabschnitt einen distalen Abschnitt (14a) und einen proximalen Abschnitt (14b) umfasst, wobei der distale Abschnitt Formgedächtnis aufweist.

3. Katheter nach Anspruch 2, wobei der distale Abschnitt (14a) gebogen ist.

4. Katheter nach Anspruch 2, wobei der proximale Abschnitt (14b) ablenkbar ist.

5. Katheter nach Anspruch 1, wobei der flexible Abschnitt die Spitzenanordnung (17) mit dem Zwischenabschnitt (14) in einem voreingestellten außeraxialen Winkel verbindet.

6. Katheter nach Anspruch 1, wobei der flexible Abschnitt wenigstens eine Stützstruktur (51) zur Minimierung der Bewegung der Spitzenanordnung (17) in einer gewählten Richtung umfasst.

7. Katheter nach Anspruch 5, wobei der außeraxiale Winkel in einer Ablenkungsrichtung des Zwischenabschnitts (14) liegt.

8. Katheter nach Anspruch 5, wobei der außeraxiale Winkel im Bereich zwischen ungefähr 2 und 180 Grad allgemein in einer Ablenkungsrichtung des Zwischenabschnitts (14) liegt.

9. Katheter nach Anspruch 5, wobei der außeraxiale Winkel im Bereich zwischen ungefähr 2 und 180 Grad allgemein entgegengesetzt zu einer Ablenkungsrichtung des Zwischenabschnitts (14) liegt.

10. Katheter nach Anspruch 1, wobei der außeraxiale Winkel im Bereich zwischen ungefähr 2 und 180 Grad allgemein in einer Ablenkungsrichtung des Zwischenabschnitts (14) liegt.

11. Katheter nach Anspruch 1, wobei der außeraxiale Winkel im Bereich zwischen ungefähr 2 und 180 Grad allgemein entgegengesetzt zu einer Ablenkungsrichtung des Zwischenabschnitts (14) liegt.

12. Katheter nach Anspruch 5, wobei die Verschiebung der Spitzenanordnung (17) zwischen der voreingestellten außeraxialen Position und einer auf der Achse liegenden Position erfolgt.

13. Katheter nach Anspruch 1, wobei die Spitzenanordnung (17) für Mapping ausgelegt ist.

14. Katheter nach Anspruch 1, wobei die Spitzenanordnung (17) für Ablation ausgelegt ist.

15. Katheter nach Anspruch 1, der ausgelegt ist, die Spitzenanordnung (17) während der Bewegung des Katheters mit dem Gewebe in Kontakt zu halten, ohne den Zwischenabschnitt (14) zu beeinträchtigen.

16. Katheter nach Anspruch 15, wobei ein abgelenkter Zwischenabschnitt (14) sich allgemein an eine Region des cavotricuspidalen Isthmus im rechten Vorhof, eine His-Region im rechten Vorhof, eine Region eines rechten Vorhofs, eine Region eines linken Vorhofs oder eine Vena cava superior anpasst.

17. Katheter nach Anspruch 15, wobei Kontakt zwischen dem Zwischenabschnitt (14) und der allgemein konvexen Region den Zwischenabschnitt zur Herzbewegung während Systole, Diastole oder Atmung synchronisiert.

18. Katheter nach Anspruch 15, wobei Kontakt zwischen dem Zwischenabschnitt (14) und der allgemein konvexen Region die Spitzenanordnung (17) allgemein stabilisiert.

19. Katheter nach Anspruch 15, wobei die Spitzenanordnung eine Ablationselektrode aufweist.

20. Katheter nach Anspruch 15, wobei die Spitzenanordnung eine Mapping-Elektrode aufweist.

21. Katheter nach Anspruch 1, wobei die Spitzenanordnung (17) eine Ablationsanordnung umfasst.

22. Katheter nach Anspruch 21, wobei die Ablationsanordnung das Folgende umfasst:
eine Vielzahl von Irrigationsöffnungen (80);
eine Spulenelektrode (82); und
eine poröse Abdeckung (84) in umgebender Beziehung zur Spulenelektrode und den Irrigationsöffnungen.

23. Katheter nach Anspruch 21, wobei die Spulenelektrode (82) eine Länge im Bereich von ungefähr 8 mm bis ungefähr 15 mm aufweist.

24. Katheter nach Anspruch 23, wobei die Spulenelektrode (82) eine Länge von ungefähr 10 mm aufweist.

25. Katheter nach Anspruch 22, wobei die poröse Abdeckung (84) expandiertes Polytetrafluorethylen umfasst.

26. Katheter nach Anspruch 21, ferner umfassend einen Temperatursensor, der in der Ablationsanordnung angebracht ist.

27. Katheter nach Anspruch 22, ferner umfassend:
eine proximale arretierende Ringelektrode (85A), die auf einer proximalen Region der Ablationsanordnung über der Spulenelektrode und der porösen Abdeckung angebracht ist; und
eine distale arretierende Ringelektrode, die auf einer distalen Region der Ablationsanordnung über der Spulenelektrode und der porösen Abdeckung angebracht ist.

28. Katheter nach Anspruch 21, ferner umfassend eine oder mehrere Ringelektroden proximal von der Ablationsanordnung.

29. Katheter nach Anspruch 21, ferner umfassend einen elektromagnetischen Sensor (72), der in der Ablationsanordnung angebracht ist.

30. Katheter nach Anspruch 21, wobei der Zwischenabschnitt (14) ferner ein Stützelement umfasst, das ein Material mit Formgedächtnis umfasst, um die vorgeformte Kurve bereitzustellen.

31. Katheter nach Anspruch 21, ferner umfassend einen Irrigationsschlauch (61), der sich in die Ablationsanordnung erstreckt.

## Revendications

1. Cathéter comprenant :
un corps de cathéter (12) allongé, flexible, tubulaire, comportant des extrémités proximale et distale ;
une section intermédiaire (14) attachée à l'extrémité distale du corps de cathéter, la section intermédiaire pouvant être courbée dans un premier plan pour ressembler à une région généralement convexe du coeur ;
un ensemble d'embout (17) ; et
une section flexible (19) raccordant l'ensemble d'embout à la section intermédiaire à un angle en dehors du plan prédéfini de telle sorte que l'ensemble d'embout s'étende dans un second plan qui est différent du premier plan, la section flexible étant conçue pour permettre un déplacement de l'ensemble d'embout vis-à-vis de l'angle prédéfini sans déplacer la section intermédiaire ;
un fil de traction (64) comportant des extrémités proximale et distale s'étendant à travers le corps de cathéter, l'extrémité distale du fil de traction étant attachée de manière fixe à l'intérieur d'une extrémité distale de la section intermédiaire ;
une poignée de commande (16) raccordée aux extrémités proximales du corps de cathéter et du fil de traction afin de déplacer le fil de traction longitudinalement par rapport au corps de cathéter, un déplacement longitudinal du fil de traction par rapport au corps de cathéter se traduisant par une courbure de la section intermédiaire ;
la section intermédiaire étant plus flexible que le corps de cathéter et la section flexible étant plus flexible que la section intermédiaire ; et
la valeur de mesure au duromètre de la section flexible n'étant pas supérieure à approximativement ½ à ¼ de la valeur de mesure au duromètre de la section intermédiaire.

2. Cathéter selon la revendication 1, dans lequel la section intermédiaire comprend une section distale (14a) et une section proximale (14b), la section distale présentant une mémoire de forme.

3. Cathéter selon la revendication 2, dans lequel la section distale (14a) est incurvée.

4. Cathéter selon la revendication 2, dans lequel la partie proximale (14b) peut être courbée.

5. Cathéter selon la revendication 1, dans lequel la section flexible raccorde l'ensemble d'embout (17) à la section intermédiaire (14) à un angle en dehors de l'axe prédéfini.

6. Cathéter selon la revendication 1, dans lequel la section flexible comprend au moins une structure de support (51) servant à minimiser le déplacement de l'ensemble d'embout (17) dans une direction sélectionnée.

7. Cathéter selon la revendication 5, dans lequel l'angle en dehors de l'axe se trouve dans une direction de courbure de la section intermédiaire (14).

8. Cathéter selon la revendication 5, dans lequel l'angle en dehors de l'axe est compris entre environ 2 et 180 degrés généralement dans une direction de courbure de la section intermédiaire (14).

9. Cathéter selon la revendication 5, dans lequel l'angle en dehors de l'axe est compris entre environ 2 et 180 degrés généralement à l'opposé d'une direction de courbure de la section intermédiaire (14).

10. Cathéter selon la revendication 1, dans lequel l'angle en dehors du plan est compris entre environ 2 et 180 degrés généralement dans une direction de courbure de la section intermédiaire (14).

11. Cathéter selon la revendication 1, dans lequel l'angle en dehors du plan est compris entre environ 2 et 180 degrés généralement à l'opposé d'une direction de courbure de la section intermédiaire (14).

12. Cathéter selon la revendication 5, dans lequel le déplacement de l'ensemble d'embout (17) se fait entre la position en dehors de l'axe prédéfinie et une position sur l'axe.

13. Cathéter selon la revendication 1, dans lequel l'ensemble d'embout (17) est configuré à des fins de cartographie.

14. Cathéter selon la revendication 1, dans lequel l'ensemble d'embout (17) est configuré à des fins d'ablation.

15. Cathéter selon la revendication 1, conçu pour maintenir l'ensemble d'embout (17) en contact avec le tissu lors du déplacement du cathéter sans affecter la section intermédiaire (14).

16. Cathéter selon la revendication 15, dans lequel une section intermédiaire courbée (14) se conforme généralement à une région de l'isthme cavo-tricuspide dans l'oreillette droite, une région du faisceau de His dans l'oreillette droite, une région d'une oreillette droite, une région d'une oreillette gauche ou une veine cave supérieure.

17. Cathéter selon la revendication 15, dans lequel un contact entre la section intermédiaire (14) et la région généralement convexe synchronise la section intermédiaire vis-à-vis des mouvements du coeur lors de la systole, la diastole ou la respiration.

18. Cathéter selon la revendication 15, dans lequel un contact entre la section intermédiaire (14) et la région généralement convexe stabilise de manière générale l'ensemble d'embout (17).

19. Cathéter selon la revendication 15, dans lequel l'ensemble d'embout comprend une électrode d'ablation.

20. Cathéter selon la revendication 15, dans lequel l'ensemble d'embout comprend une électrode de cartographie.

21. Cathéter selon la revendication 1, dans lequel l'ensemble d'embout (17) comprend un ensemble d'ablation.

22. Cathéter selon la revendication 21, dans lequel l'ensemble d'ablation comprend :
une pluralité d'orifices d'irrigation (80) ;
un fil-électrode (82) ; et
un revêtement poreux (84) entourant le fil-électrode et les orifices d'irrigation.

23. Cathéter selon la revendication 21, dans lequel le fil-électrode (82) présente une longueur allant d'environ 8 mm à environ 15 mm.

24. Cathéter selon la revendication 23, dans lequel le fil-électrode (82) présente une longueur d'environ 10 mm.

25. Cathéter selon la revendication 22, dans lequel le revêtement poreux (84) comprend du polytétrafluoroéthylène expansé.

26. Cathéter selon la revendication 21, comprenant en outre un capteur de température installé dans l'ensemble d'ablation.

27. Cathéter selon la revendication 22, comprenant en outre :
une électrode annulaire de blocage proximale (85A) installée sur une région proximale de l'ensemble d'ablation par-dessus le fil-électrode et le revêtement poreux ; et
une électrode annulaire de blocage distale installée sur une région distale de l'ensemble d'ablation par-dessus le fil-électrode et le revêtement poreux.

28. Cathéter selon la revendication 21, comprenant en outre une ou plusieurs électrodes annulaires en position proximale vis-à-vis de l'ensemble d'ablation.

29. Cathéter selon la revendication 21, comprenant en outre un capteur électromagnétique (72) installé dans l'ensemble d'ablation.

30. Cathéter selon la revendication 21, dans lequel la section intermédiaire (14) comprend en outre un élément de support comprenant un matériau présentant une mémoire de forme afin de produire la courbe préformée.

31. Cathéter selon la revendication 21, comprenant en outre un tube d'irrigation (61) s'étendant dans l'ensemble d'ablation.
